# EUROPEAN PATENT APPLICATION

(11) **EP 4 230 738 A1**
(43) Date of publication of application: **23.08.2023**
(21) Application number: 21872719.6
(22) Date of filing: 12.08.2021
(51) Int. Cl.: C12N 15/10, C12N 9/12, C12N 9/22, C12N 15/113, C12N 15/63

(54) **PRIME EDITING-BASED GENE EDITING COMPOSITION WITH ENHANCED EDITING EFFICIENCY AND USE THEREOF**

(30) Priority: 24.09.2020 KR 20200123432
(71) Applicant: Korea University Research and Business Foundation, Seoul 02841 (KR); Seoul National University R & DB Foundation, Seoul 08826 (KR); Korea Mouse Phenotyping Center, Seoul 08826 (KR)
(72) Inventor: KIM, Kyoungmi, Seoul 03024 (KR); PARK, Soo Ji, Pyeongtaek-si, Gyeonggi-do 17841 (KR); JEONG, Tae-Yeong, Hanam-si, Gyeonggi-do 12914 (KR); SHIN, Seung Kyun, Seoul 02843 (KR); SEONG, Je Kyung, Seoul 07986 (KR)
(74) Representative: Henderson, Helen Lee
(86) International application number: PCT/KR2021/010740
(87) International publication number: WO 2022/065689

(57) **Abstract**

The present invention relates to a prime editing-based gene editing composition with enhanced editing efficiency, a method for gene editing by using the composition, a gene editing kit, and a method for construction of a gene-modified mammal. The prime editor developed in the present invention exhibits remarkably enhanced genome editing efficiency and target specificity and mutant animal models constructed by using same were observed to perform mutation transmission to the next generation and a phenotype change in the next generation. Thus, the enhanced prime editor or a gene editing composition comprising same can find advantageous applications in various purposes, such as the construction and research of humanized animal models, the genetic engineering technical field, and therapeutic means for genetic diseases.

## Description

### Technical Field

The present disclosure relates to a prime editing-based gene editing composition with enhanced gene editing efficiency, a method for gene editing using the composition, a gene editing kit, and a method for construction of a gene-modified mammal.

### Background Art

A CRISPR-CAS system has been advanced into a variety of advanced genome editing tools, such as nucleases, base editors, and transposases capable of effectively inducing a desired target mutation. In particular, a cytosine base editor (CBE) and an adenine base editor (ABE), which were developed based on the CRISPR system, may substitute C.G and A.T with T.A and G.C in various organisms including mice. In addition, through a recent study, CGBE1 has been reported as C-to-G base editors capable of base editing from C to G in human cells. However, it is still difficult to generate an accurate target mutation, such as insertion, substitution or cleavage of one or more bases due to the limitations of gene editing by low efficiency of intracellular homology-directed repair (HDR).

A prime editor (PE), which is a new concept of genome editing tool developed at the demand, is a fusion protein consisting of CAS9 nickase-H840A and reverse transcriptase (RT), which is modified to cleave only one of DNA double strands. The PE requires prime editing guide RNA (pegRNA) which is a new type of guide RNA encoding a desired editing sequence. The pegRNA has a RT template of reverse transcriptase comprising a primer binding site (PBS) complementary to a non-target strand of a target gene and a base sequence to be edited. The CAS9 nickase of the prime editor cleaves the non-target strand of the target gene and the reverse transcriptase synthesizes a new DNA strand including a base sequence edited based on the RT template strand of the pegRNA, and then the existing DNA sequence is removed by an intracellular recovery process and replaced with the newly synthesized edited DNA base sequence. Through this sophisticated genome editing system, it is possible to generate a target mutation, including a substitution from a base to a base, and a small size of insertion and deletion without cleavage of double-stranded DNA or donating DNA. However, the prime editor also has a problem of off-target which has a lower frequency than the base editor, and particularly, there is a problem in which accurate editing is difficult due to the insertion and deletion of additional base sequences occurring by reverse transcriptase. Moreover, in addition the problems, the currently developed base editors and prime editors have a limit of very low efficiency. For example, the most efficient PE3 among the prime editors is only 20 to 50% of genetic editing efficiency.

Intracellular genetic materials in the cells of living things including humans are always exposed to mutations, and the accumulation of mutations leads to various mutant phenotypes, and some thereof are associated with a disease. Therefore, sophisticated gene editing technology using prime editing may be used as a tool for the treatment of genetic diseases, and may be widely used for identifying pathogenic mutant factors and studying disease mechanisms. Therefore, it is necessary to develop a prime editor that is more sophisticated than the known prime editors and has improved editing efficiency and a gene editing technology using the same.

### [Related Art Document]

### [Non-Patent Document]

(Non-Patent Document 0001) Int J Mol Sci. 2020 Aug 28;21(7):6240

### Disclosure of the Invention

### Technical Goals

In order to overcome the problems of prime editing-based gene editing, the present inventors developed an improved prime editor using proxymal dead sgRNA (dsgRNA) and/or chromatin-modulating peptides (CMPs), confirmed significantly enhanced genome editing efficiency thereof, target specificity, and the like through specific experiments, and then completed the present disclosure.

Accordingly, an object of the present disclosure is to provide a prime editing-based gene editing composition with enhanced gene editing efficiency.

Another object of the present disclosure is to provide a method for gene editing using the gene editing composition.

Yet another object of the present disclosure is to provide a gene editing kit comprising the gene editing composition.

Still another object of the present disclosure is to provide a method for construction of a gene-modified mammal other than human using the gene editing composition.

However, technical objects of the present disclosure are not limited to the aforementioned purpose and other objects which are not mentioned may be clearly understood to those skilled in the art from the following description.

### Technical Solutions

In order to achieve the object of the present disclosure, the present disclosure provides a gene editing composition including (a) a fusion protein or a nucleic acid encoding the fusion protein including i) a CRISPR/Cas9 protein or a variant thereof, and ii) a reverse transcriptase or a variant thereof; and
(b) a guide RNA or a nucleic acid encoding the guide RNA,
in which the guide RNA includes prime editing guide RNA (pegRNA) and dead single guide RNA (dsgRNA), and the dsgRNA is 10 to 20 bp in length.

In an embodiment of the present disclosure, the dsgRNA may bind to a location of 5 to 70 nucleotides away from the pegRNA binding site to increase chromatin accessibility of the fusion protein.

In another embodiment of the present disclosure, the gene editing composition may further include single guide RNA (sgRNA) that complementarily binds to a non-target DNA strand to induce cleavage of a target DNA strand.

In addition, the present disclosure provides a gene editing composition including (a) a fusion protein or a nucleic acid encoding the fusion protein including i) a CRISPR/Cas9 protein or a variant thereof, ii) a reverse transcriptase or a variant thereof, and iii) chromatin-modulating peptides; and
(b) a guide RNA or a nucleic acid encoding the guide RNA,
in which the guide RNA includes prime editing guide RNA (pegRNA) and dead single guide RNA (dsgRNA).

In one embodiment of the present disclosure, the chromatin-modulating peptide may be a high-mobility group nucleosome binding domain 1 (HN1), a histone H1 central globular domain (H1G), or a combination thereof.

In another embodiment of the present disclosure, the chromatin-modulating peptides may be linked to the CRISPR/Cas9 protein or reverse transcriptase directly by a chemical bond, indirectly by a linker, or a combination thereof.

In another embodiment of the present disclosure, the fusion protein may consist of N-terminus-[HN1]-[Cas9]-[H1G]-[reverse transcriptase]-C-terminus; or N-terminus-[HN1]-[Cas9]-[reverse transcriptase]-[H1G]-C terminus.

In another embodiment of the present disclosure, the fusion protein may further include a nuclear localization signal (NLS) sequence at the N-terminus and C-terminus, respectively.

In yet another embodiment of the present disclosure, the CRISPR/Cas9 protein variant may be nickase.

In yet another embodiment of the present disclosure, in the CRISPR/Cas9 protein variant, either a RuvC domain or a HNH domain may be deactivated.

In yet another embodiment of the present disclosure, the reverse transcriptase or the variant thereof may be derived from a Moloney murine leukemia virus (M-MLV).

In yet another embodiment of the present disclosure, the fusion protein may consist of an amino acid sequence represented by SEQ ID NO: 1 or SEQ ID NO: 2.

In yet another embodiment of the present disclosure, the dsgRNA may consist of 10 to 20 nucleotides in length.

In yet another embodiment of the present disclosure, the dsgRNA may bind to a location of 5 to 70 nucleotides away from the pegRNA binding site to increase chromatin accessibility of the fusion protein.

In yet another embodiment of the present disclosure, the gene editing composition may enhance the gene editing efficiency and target specificity.

According to another embodiment of the present disclosure, there is provided a gene editing composition including (a) a protein or a nucleic acid encoding the protein including i) a CRISPR/Cas9 protein or a variant thereof; ii) a reverse transcriptase or a variant thereof; and iii) chromatin-modulating peptides; and
(b) a guide RNA or a nucleic acid encoding the guide RNA,
in which the guide RNA includes prime editing guide RNA (pegRNA) and dead single guide RNA (dsgRNA).

In an embodiment of the present disclosure, i) the CRISPR/Cas9 protein or the variant thereof; and ii) the reverse transcriptase or the variant thereof may be fused to be transmitted into cells or i) and ii) may be separately expressed to be transmitted into cells in the form of plasmid DNA, RNA, or protein.

According to yet another embodiment of the present disclosure, there is provided a method for gene editing including bringing the gene editing composition into contact with a target region including a target nucleic acid sequence *in vitro* or ex vivo.

According to still another embodiment of the present disclosure, there is provided a gene editing kit including the gene editing composition.

According to still another embodiment of the present disclosure, there is provided a method for construction of a gene-modified mammal other than human including: obtaining a gene-modified mammal cell by introducing the gene editing composition into a mammal cell other than human; and
transplanting the obtained gene-modified mammal cell into the fallopian tube of a mammal foster mother other than human.

As one embodiment of the present disclosure, the mammal cell may be a mammal embryonic cell.

### Effects

According to the embodiments of the present disclosure, a prime editor with enhanced performance was developed using dead sgRNA (dsgRNA) and/or chromatin-modulating peptides (CMPs), significantly enhanced genome editing efficiency and target specificity of the prime editor were confirmed, and mutant animal models of the target gene were constructed to confirm transmission of the mutation to the next generation, phenotypic changes, and the like. Therefore, the gene editing composition including the enhanced prime editor according to the present disclosure can be usefully used for various applications, such as the construction and research of humanized animal models, a field of genetic engineering technology, and the treatment of genetic diseases.

### Brief Description of Drawings

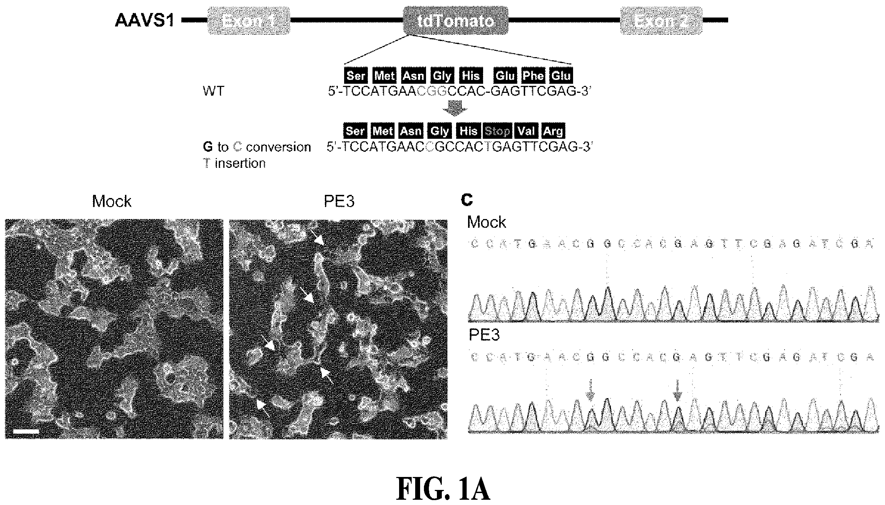

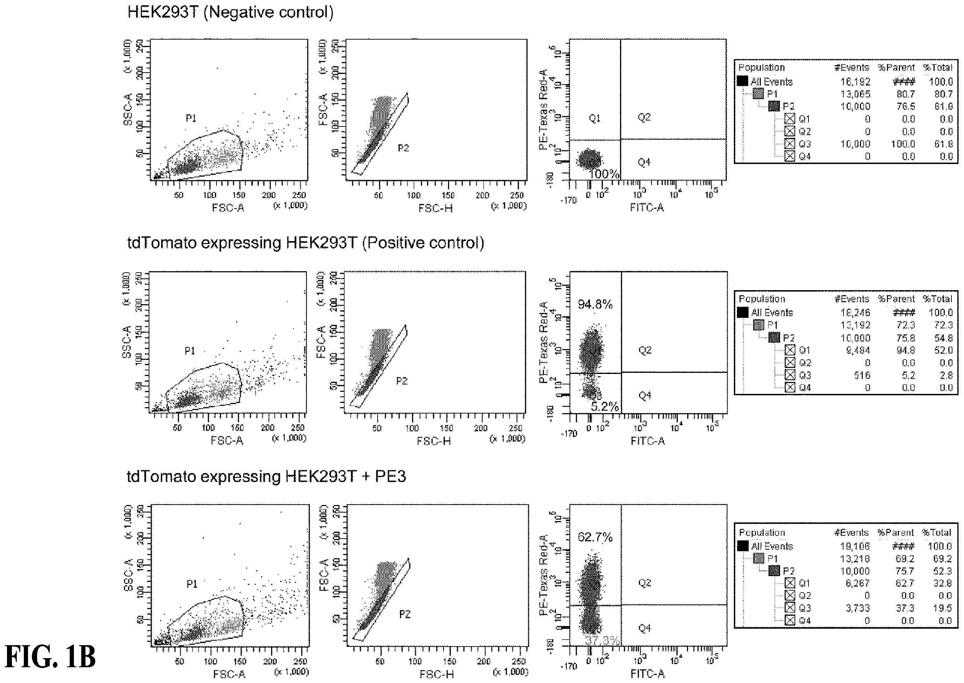

FIG. 1 illustrates results of verifying a prime editing system in a mammal genome, in which FIG. 1A is a schematic diagram illustrating a design of a target mutation for a tdTomato gene in an AAVS1 gene of reporter HEK293T cells expressing tdTomato, and shows microscopic images and Sanger sequencing chromatograms showing the editing efficiency of PE3 in the cells, and FIG. 1B illustrates results of counting tdTomato-negative and tdTomato-positive cells by FACS after culturing cells with or without PE3 for 11 days.

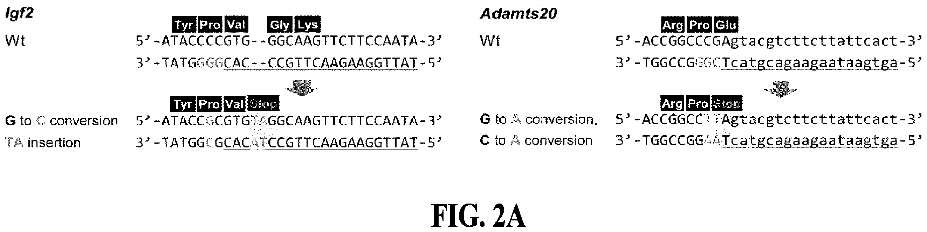

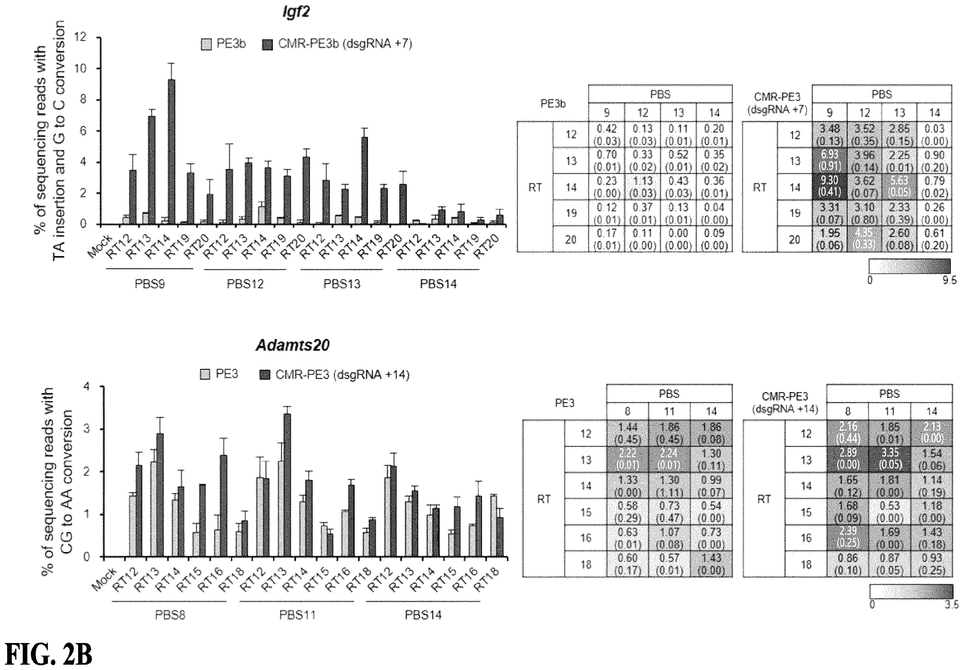

FIG. 2 illustrates results of optimizing prime editing efficiency using pegRNAs and dsgRNA of various lengths at target sites of mouse genes *Igf2* and *Adamts20,* in which FIG. 2A is a schematic diagram illustrating a target mutation design in each of two gene targets, and FIG. 2B illustrates a result of comparing the prime editing efficiency of PE3 and CMR-PE3 according to various combinations of primer binding sites (PBS) and reverse transcriptase template lengths in mouse-derived NIH/3T3 cells.

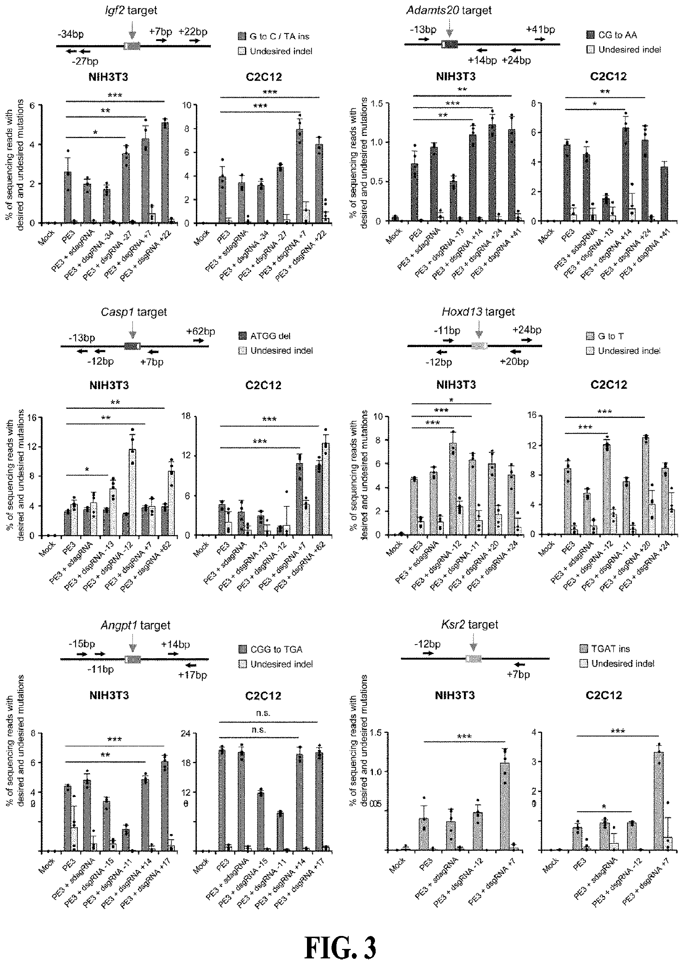

FIG. 3 illustrates results of comparing and analyzing prime editing efficiency of PE3 according to whether dsgRNA is used for targets of respective genes *Igf2, Adamts20, Caspl, Hoxd13, Angptl* and *Ksr2* in sequence in mouse-derived NIH/3T3 or C2C12 cells (PE3, PE3 + dsgRNA).

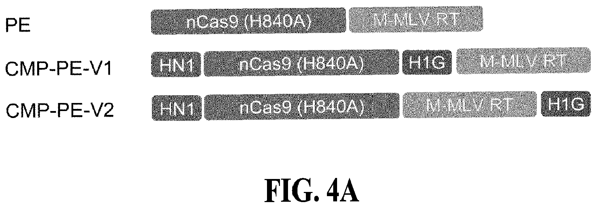

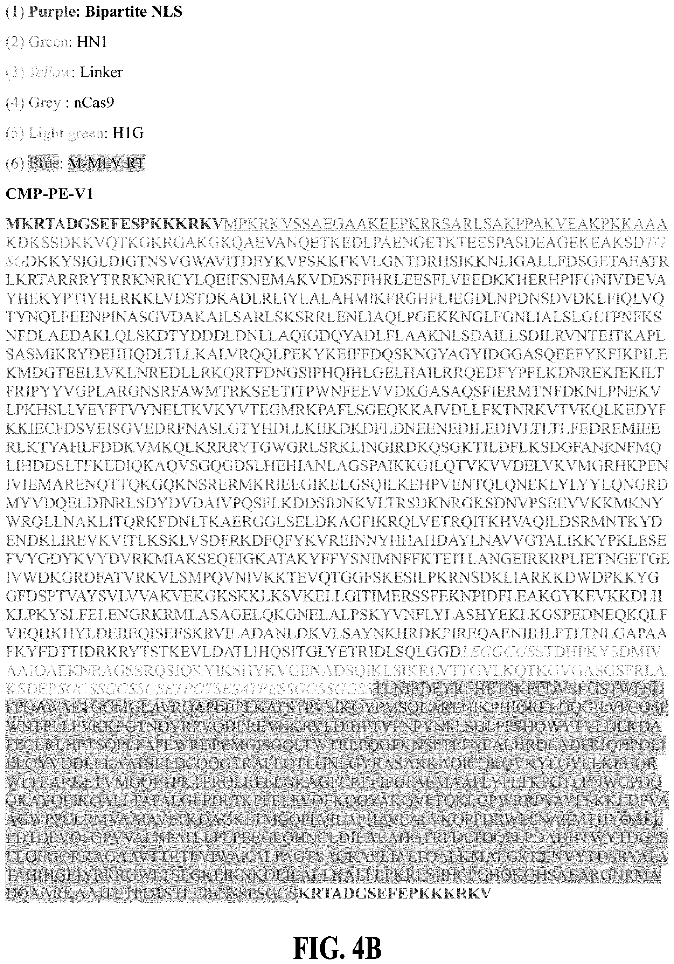

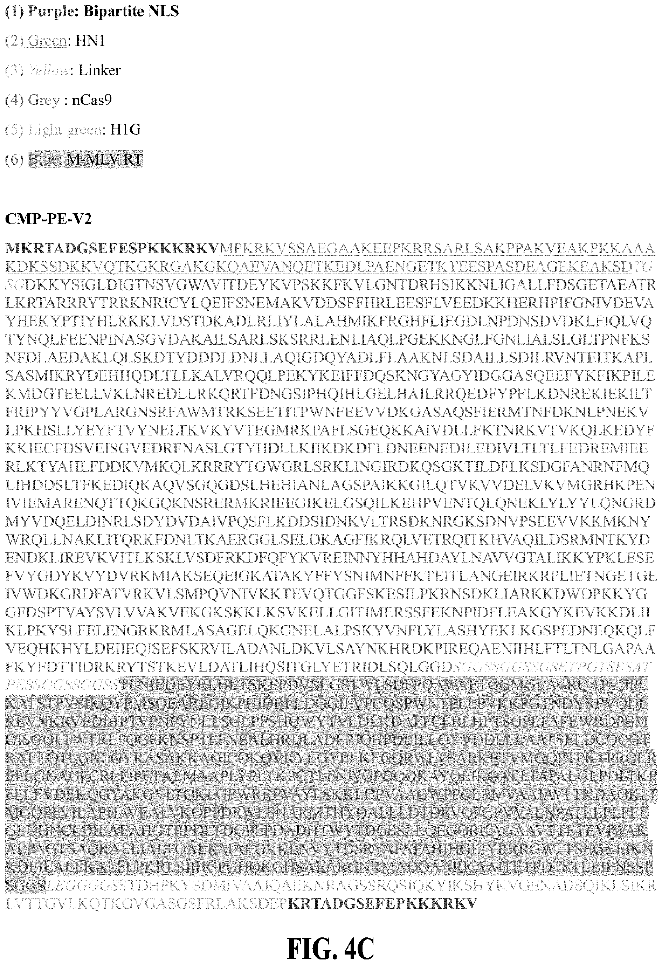

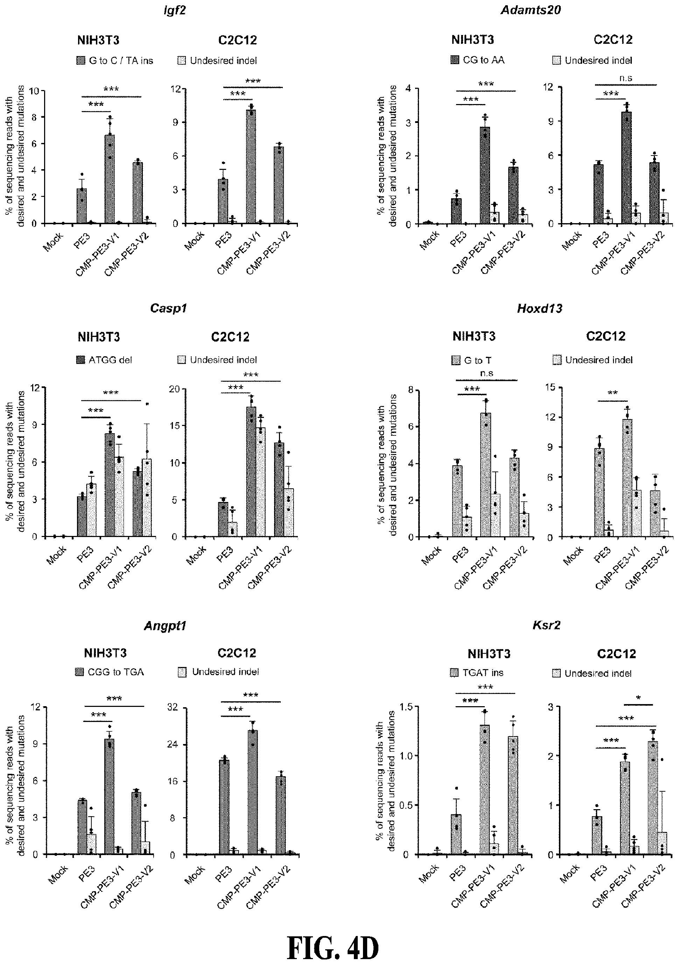

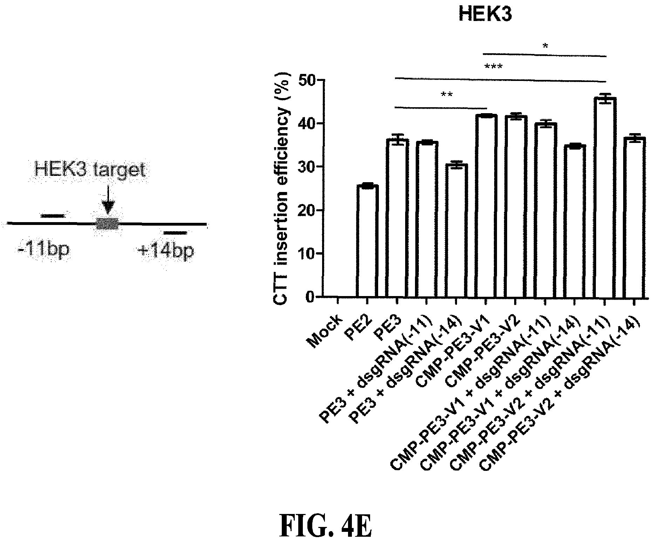

FIG. 4 illustrates results of confirming an effect of enhancing prime editing efficiency by binding of chromatin-modulating peptides (CMPs), in which FIG. 4A illustrates a structure of two types of fusion proteins (CMP-PE-V1, CMP-PE-V2) to which CMP is bound, FIGS. 4B and 4C illustrate amino acid sequences of CMP-PE-V1 and CMP-PE-V2 by component, respectively, and FIG. 4D illustrates results of comparing and analyzing prime editing efficiency according to CMP (HN1, H1G) binding to targets in genes *Igf2, Adamts20, Casp1, Hoxd13, Angpt1* and *Ksr2* in NIH/3T3 or C2C12 cells. FIG. 4E illustrates a result of comparing and analyzing prime editing efficiency in HEK293T cells as human cells, targeting a HEK3 sequence.

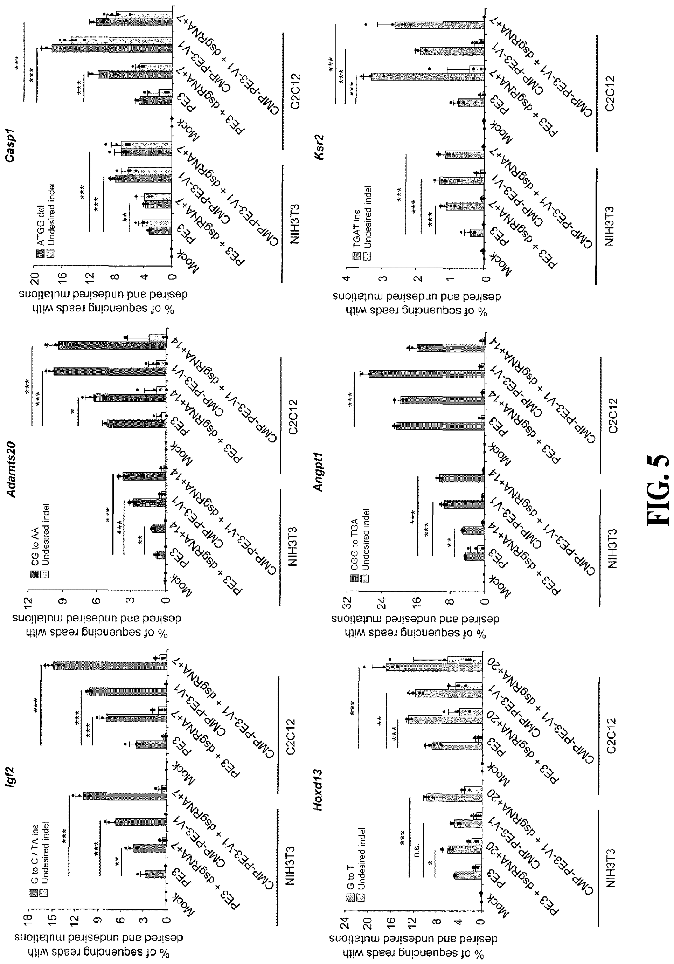

FIG. 5 illustrates results of comparing and analyzing prime editing efficiency of PE3 + dsgRNA using PE3 and dsgRNA, CMP-PE3-V1 bound with CMP, and CMP-PE3-V1 + dsgRNA using CMP and dsgRNA for targets of each of genes *Igf2, Adamts20, Caspl, Hoxd13, Angptl* and *Ksr2* in NIH/3T3 and C2C12 cells.

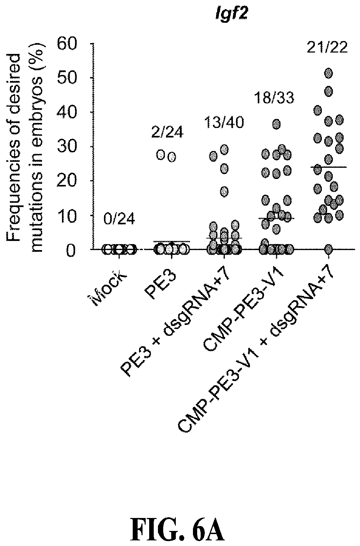

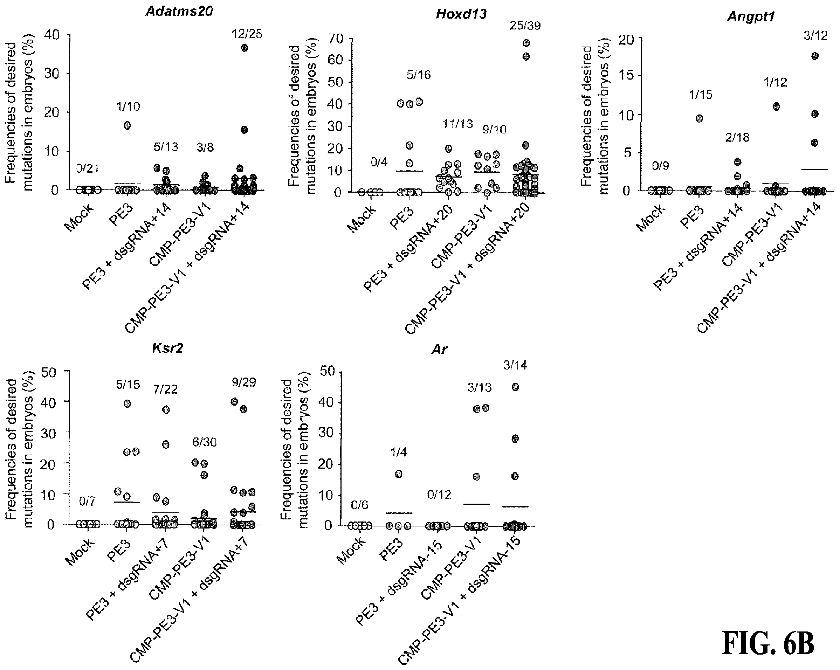

FIG. 6 illustrates results of analyzing frequencies of target mutations in mouse embryos injected with each of prime editing systems of PE3, PE3 + dsgRNA, CMP-PE3-V1 and CMP-PE3-V1 + dsgRNA, in which FIG. 6A illustrates results in *Igf2,* and FIG. 6B illustrates results in *Adamts20, Hoxd13, Angpt1*, *Ksr2* and *Ar*.

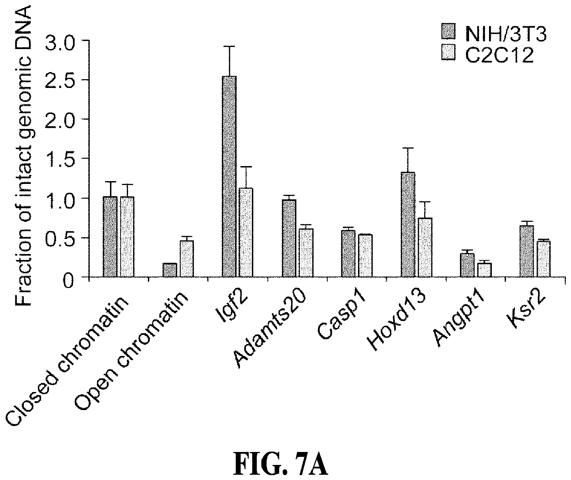

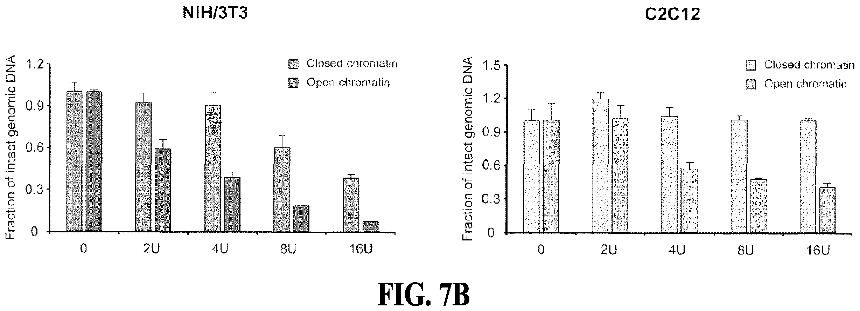

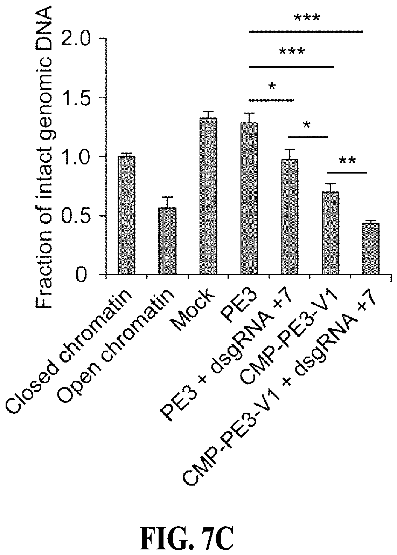

FIG. 7 illustrates results of measuring the fraction of intact genomic DNA through real-time qPCR after DNase I digestion analysis in NIH/3T3 and C2C12 cells, respectively. FIG. 7A illustrates a result of measuring the fraction of intact genomic DNA at *Igf2, Adamts20, Casp1, Hoxd13, Angpt1* and *Ksr2* target loci in each of the cells, FIG. 7B illustrates a result of treating the two cell lines differently with 2 to 16 U of DNase I and measuring the fraction of intact genomic DNA, and FIG. 7C illustrates a result of measuring the fraction of intact genomic DNA at an *Igf2* target locus after transfecting C2C12 cells with plasmids encoding PE3, PE3 + dsgRNA, CMP-PE3-V1, or CMP-PE3-V1 + dsgRNA.

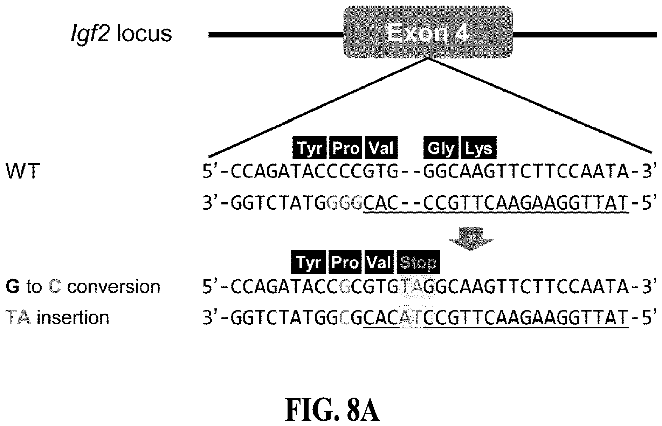

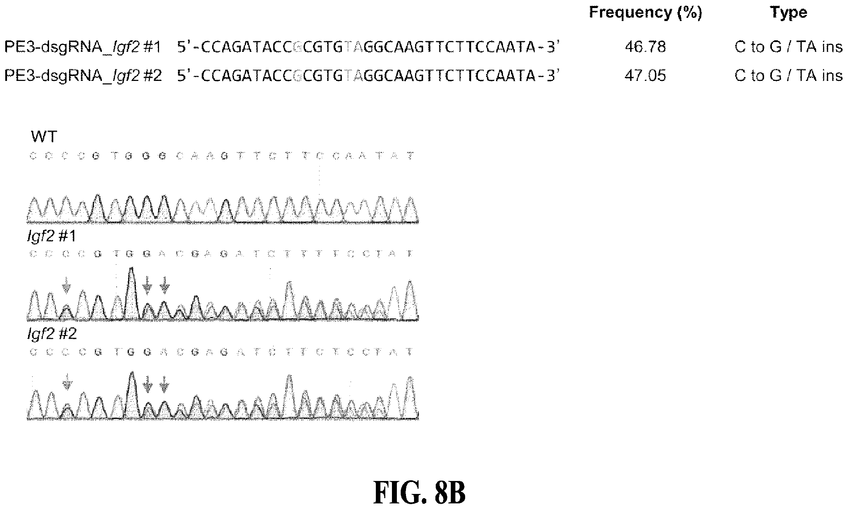

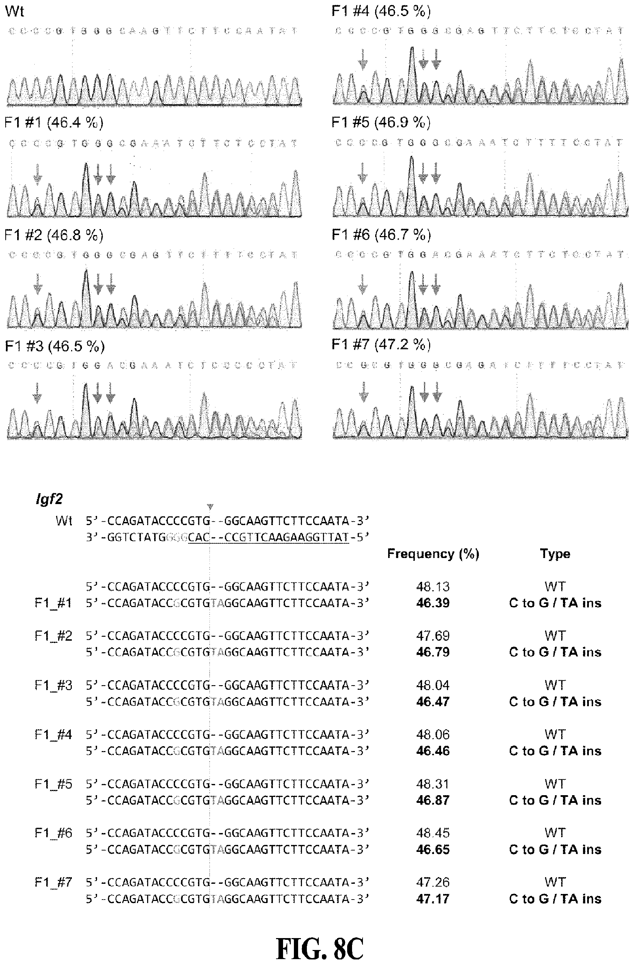

FIG. 8 illustrates results of inducing target mutations in mice mediated by PE3 using dsgRNA. FIG. 8A is a schematic diagram illustrating a design of target mutations in exon4 of a *Igf2* gene, FIG. 8B illustrates genotyping and Sanger sequencing chromatogram results of two mice with target mutations (G to C substitution and TA insertion) induced by PE3 using proximal dsgRNA +7 in *Igf2,* and FIG. 8C illustrates a result of confirming target mutations through Sanger sequencing and genotyping for 7 mice of the F1 generation of the constructed *Igf2* mutant mice.

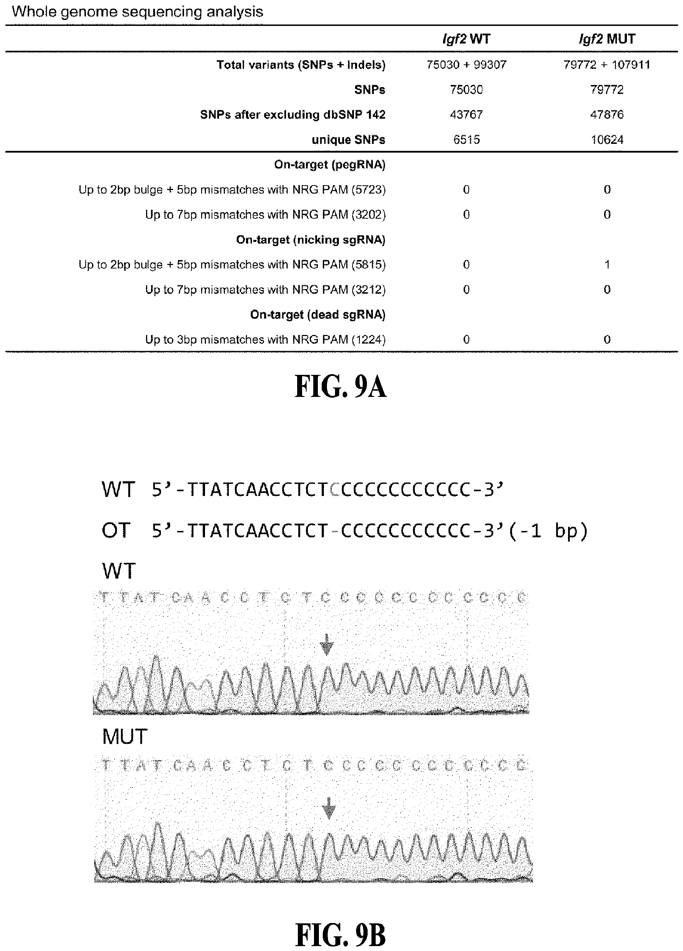

FIG. 9 illustrates results of analyzing an off-target effect of the prime editor according to the present disclosure. FIG. 9A illustrates a result of measuring insertion/deletion (indel) frequencies at potential off-target sites of pegRNA and nsgRNA used for generating *Igf2* target mutations in a wild-type and mutant *Igf2* #1 and *Igf2* #2 mice constructed in FIG. 8, FIG. 9B illustrates a result of comparing whole genome sequencing performed on a wild-type (*Igf2* WT) and *Igf2* #1 mice, and FIG. 9C illustrates a result of comparing base sequences for potential off-target (OT) locations and showing a Sanger sequencing chromatogram.

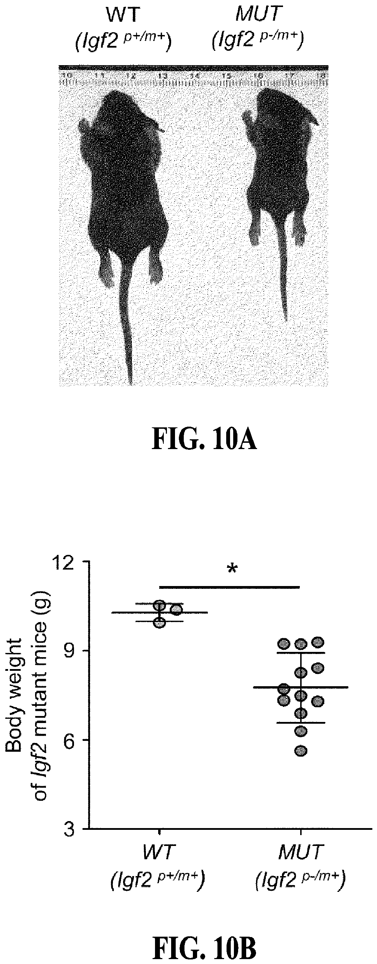

FIG. 10 illustrates results of confirming phenotypes of *Igf2* mutant mice, in which FIG. 10A shows images of dwarfism phenotypes of *Igf2* mutant mice (MUT (*Igf2*^{p-/m+})) obtained by mating *Igf2*^{p+/m-} males (F1) with wild-type female mice, and FIG. 10B illustrates a result of measuring and comparing body weights of the *Igf2* mutant mice and the *Igf2* wild-type mice.

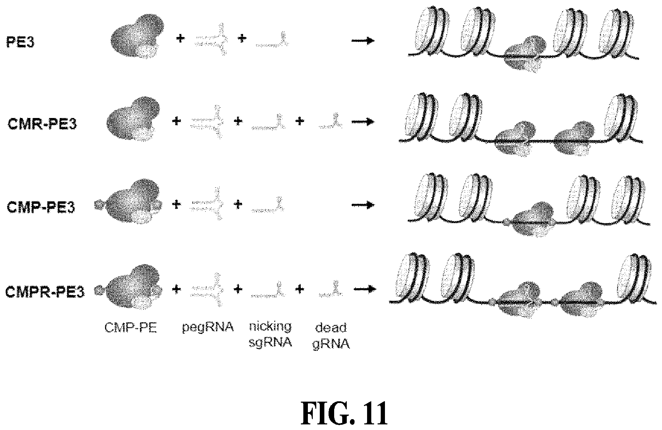

FIG. 11 is a diagram illustrating configurations and mechanisms of three types of prime editing systems with enhanced editing efficiency according to the present disclosure compared with conventionally known PE3.

### Best Mode for Carrying Out the Invention

The present inventors have developed a prime editor with improved problems in the related art using proxymal dsgRNA (dead sgRNA) and/or chromatin-modulating peptides (CMPs), confirmed excellent editing efficiency and target specificity of the prime editor, and then completed the present disclosure.

The present disclosure provides a gene editing composition including (a) a fusion protein or a nucleic acid encoding the fusion protein, including i) a CRISPR/Cas9 protein or a variant thereof, and ii) a reverse transcriptase or a variant thereof; and
(b) a guide RNA or a nucleic acid encoding the guide RNA,
in which the guide RNA includes prime editing guide RNA (pegRNA) and dead single guide RNA (dsgRNA), and the dsgRNA is 10 to 20 nt in length.

The gene editing composition may further include single guide RNA (sgRNA) that complementarily binds to a non-target DNA strand to induce cleavage of a target DNA strand, and preferably, the guide RNA may refer to nicking sgRNA.

The term "gene editing" used herein may be used as the same meaning as gene editing, genome editing, and the like. The gene editing refers to mutations (substitution, insertion or deletion) that cause mutations of one or more bases at a target site in a target gene. Preferably, the gene editing may not involve double-stranded DNA cleavage of the target gene, and more specifically, may be performed via prime editing.

In one embodiment of the present disclosure, mutation or gene editing that causes mutations for the one or more bases generates a stop codon at the target site, or generates a codon encoding an amino acid different from a wild type to knock-out the target gene. Alternatively, the mutation or gene editing may be various forms, such as knocking-out a gene or editing a genetic mutation by changing an initiation codon to another amino acid; knocking-out a gene or editing a genetic mutation by frameshift due to insertion or deletion; introducing mutations into a non-coding DNA sequence that do not produce proteins; changing DNA with a sequence different from a wild type causing a disease to the same sequence as the wild type, or the like, but is not limited thereto.

The term "base sequence" used herein refers to a nucleotide sequence including the corresponding bases, and may be used in the same meaning as a nucleotide sequence, a nucleic acid sequence, or a DNA sequence.

In the present disclosure, the 'target gene' refers to a gene targeted for gene editing, and the 'target site or target region' refers to a site where gene editing or revising occurs by target-specific nuclease within a target gene. In one example, when the target-specific nuclease includes RNA guided engineered nuclease (RGEN), the target site may be located adjacent to a 5' end and/or 3' end of a sequence (PAM sequence) recognized by the RGEN in the target gene.

In addition, the present disclosure provides a gene editing composition including (a) a fusion protein or a nucleic acid encoding the fusion protein, including i) a CRISPR/Cas9 protein or a variant thereof, ii) a reverse transcriptase or a variant thereof, and iii) chromatin-modulating peptides; and
(b) a guide RNA or a nucleic acid encoding the guide RNA,
in which the guide RNA includes prime editing guide RNA (pegRNA) and dead single guide RNA (dsgRNA).

The gene editing composition may further include single guide RNA (sgRNA) that complementarily binds to a non-target DNA strand to induce cleavage of a target DNA strand, and preferably, the guide RNA may refer to nicking sgRNA in the present disclosure.

In the present disclosure, the chromatin-modulating peptides refer to chromosomal proteins or fragments thereof that interact with nucleosomes and/or chromosomal proteins to facilitate nucleosome rearrangement and/or chromatin remodeling. More specifically, the chromatin-modulating peptide may be a high-mobility group nucleosome binding domain 1 (HN1) or a fragment thereof, a histone H1 central globular domain (H1G) or a fragment thereof, or a combination thereof, but is not limited thereto.

The high-mobility group nucleosome binding domain (HMGN) is a chromosomal protein that modulates the structure and function of chromatin, and the histone H1 central globular domain (H1G) is a domain that constitutes histone H1, also referred to as `linker histone'. It is known that the histone H1 modulates a compaction state of a nucleosome array and affects its shape, and the central globular domain binds near an entry/exit site of the linker DNA on the nucleosome.

The chromatin-modulating peptides may be linked to the CRISPR/Cas9 protein or the reverse transcriptase directly by a chemical bond, indirectly by a linker, or in combination thereof. Specifically, at least one chromatin-modulating peptide may be linked to an N-terminus, a C-terminus, and/or an internal location of the CRISPR/Cas9 protein. Preferably, the fusion protein of the present disclosure includes two chromatin-modulating peptides linked to the CRISPR/Cas9 protein or the reverse transcriptase. More preferably, in the fusion protein, the HMGN1 (HN1) may be linked to the N-terminus of the Cas9 protein or the variant thereof, and the histone H1 central globular domain (H1G) may be linked to the C-terminus of the Cas9 protein or the variant thereof or to the C-terminus of the reverse transcriptase. Most preferably, the fusion protein may consist of i) N-terminus-[HN1]-[Cas9]-[H1G]-[reverse transcriptase]-C-terminus; or ii) N-terminus-[HNl]-[Cas9]-[reverse transcriptase]-[H1G]-C terminus.

In the present disclosure, the fusion protein may further include at least one nuclear localization signal, at least one cell-penetration domain, at least one marker domain, or a combination thereof, preferably further include a nuclear localization signal (NLS) sequence to the N-terminus and the C-terminus, respectively, but is not limited thereto.

The fusion protein according to the present disclosure may consist of an amino acid sequence represented by SEQ ID NO: 1 or SEQ ID NO: 2. At this time, the fusion protein may include an amino acid sequence having 70% or more, preferably 80% or more, more preferably 90% or more, and most preferably 95%, 96%, 97%, 98%, and 99% or more of sequence homology with the amino acid sequence represented by SEQ ID NO: 1 or SEQ ID NO: 2.

In the present disclosure, the 'CRISPR associated protein 9 (Cas9)' is a protein that plays an important role in the immunological defense of specific bacteria against DNA viruses, and widely used in genetic engineering applications, but may be applied to modify the genome of a cell because the main function of the protein is to cleave DNA. Specifically, CRISPR/Cas9 recognizes, cleaves, and edits a specific base sequence to be used as 3G gene scissors, and is useful for simply, quickly, and efficiently performing an operation of inserting a specific gene into a target site in the genome or stopping the activity of the specific gene. The Cas9 protein or gene information may be obtained from a known database such as GenBank of the National Center for Biotechnology Information (NCBI), but is not limited thereto. In addition, the Cas9 protein may appropriately link an additional domain by those skilled in the art depending on its purpose. In the present disclosure, the Cas9 protein may include not only wild-type Cas9 but also Cas9 variants as long as the protein has a function of nuclease for gene editing.

In the present disclosure, the Cas9 variant may be mutated to lose the activity of endonuclease for cleaving DNA double strands. For example, the Cas9 variant may be at least one selected from a Cas9 protein mutated to lose endonuclease activity and have nickase activity and a Cas9 protein mutated to lose both the endonuclease activity and the nickase activity, preferably Cas9 nickase.

The Cas9 nickase may be deactivated by a mutation in a catalytically active domain (e.g., RuvC or HNH domain of Cas9) of the nuclease. Specifically, the Cas9 nickase may include a mutation in which one or more selected from the group consisting of aspartic acid (D10) at position 10, glutamic acid (E762) at position 762, histidine (H840) at position 840, asparagine (N854) at position 854, asparagine (N863) at position 863 and aspartic acid (D986) at position 986 are substituted with any other amino acids. Preferably, the Cas9 nickase of the present disclosure may include a mutation in which histidine at position 840 is substituted with alanine (H840A), but is not limited thereto.

The origin of the Cas9 protein or the variant thereof is not limited, and nonlimiting examples may be derived from *Streptococcus pyogenes, Francisella novicida, Streptococcus thermophilus, Legionella pneumophila, Listeria innocua,* or *Streptococcus mutans.*

The Cas9 protein or the variant thereof may be isolated from microorganisms or occur artificially or non-naturally, such as by a recombinant method or a synthetic method. The Cas9 may be used in the form of pre-transcribed mRNA or pre-produced protein *in vitro,* or contained in a recombinant vector for expression in a target cell or *in vivo.* In one embodiment, the Cas9 may be a recombinant protein produced by recombinant DNA (rDNA). The recombinant DNA refers to DNA molecules artificially produced by genetic recombination methods such as molecular cloning to contain heterologous or homologous genetic materials obtained from various organisms.

In the present disclosure, the 'reverse transcriptase' refers to an enzyme having the ability to synthesize DNA using RNA as a template. In the present disclosure, the reverse transcriptase may include not only a wild-type reverse transcriptase, but also a variant of the reverse transcriptase as long as the reverse transcriptase has a function of synthesizing DNA using RNA as a template, and the reverse transcriptase or the variant thereof may be preferably derived from Moloney murine leukemia virus (M-MLV), but is not limited thereto.

The term "guide RNA" as used herein refers to RNA comprising a targeting sequence hybridizable with a specific base sequence (target sequence) within a target site in a target gene, and serves to bind to a nuclease protein such as Cas *in vitro* or *in vivo* (or cell) to be guided to a target gene (or target site).

The guide RNA may be appropriately selected depending on a type of nuclease to form a complex and/or a microorganism derived therefrom. For example, the guide RNA of the present disclosure may be pegRNA, dead sgRNA or nicking sgRNA.

The guide RNA may include a spacer region (also referred to as target DNA recognition sequence, base pairing region, etc.), which is a portion having a complementary sequence (targeting sequence) to a target sequence in a target gene (target site), and a hairpin structure for binding the Cas9 protein. More specifically, the guide RNA may include a region having a complementary sequence to a target sequence in a target gene, a hairpin structure for binding the Cas9 protein, and a terminator sequence. Additionally, the pegRNA has a RT template of reverse transcriptase comprising a primer binding site (PBS) complementary to a non-target strand of a target gene and a base sequence to be edited.

The targeting sequence of the guide RNA capable of hybridizing with the target sequence of the guide RNA refers to a nucleotide sequence having at least 50%, at least 60%, at least 70%, at least 80%, at least 90%, at least 95%, at least 99%, or 100% of sequence complementarity with a nucleotide sequence of a DNA strand (that is, a DNA strand on which a PAM sequence (5'-NGG-3' (N is A, T, G, or C)) is located) or its complementary strand at which the target sequence is located, and is capable of complementary binding with the nucleotide sequence of the complementary strand.

The guide RNA may be used in the form of RNA (or included in the composition) or used in the form of a plasmid (or included in the composition) containing DNA encoding the RNA.

In the present disclosure, the dsgRNA may consist of 10 to 20 nt (nucleotides) in length, preferably 11 to 19 nt, 12 to 18 nt, 13 to 17 nt, 13 to 16 nt, and most preferably 14 to 15 nt in length, but is not limited thereto.

In addition, the dsgRNA may bind to a location of 5 to 70 nt (nucleotides), preferably 6 to 65 nt, and most preferably 7 to 62 nt away from the pegRNA binding site, preferably the spacer site of the pegRNA, to increase chromatin accessibility of the fusion protein.

The term "chromatin accessibility" as used herein refers to a physical compaction level of chromatin which is a complex mainly formed by DNA consisting of histone, a transcription factor (TF), chromatin-modifying enzymes, and chromatin-remodeling complexes and related proteins. An eukaryotic genome is generally compacted into a nucleosome containing ~147 bp of DNA surrounding a histone octamer, but the occupancy of nucleosomes is not uniform in the genome and varies according to tissue and cell types. The nucleosomes are depleted at a genomic location where cis regulatory elements (enhancers and promoters) interacting with a transcriptional regulator (ex. transcription factor) are usually present to produce an accessible chromatin. In regard to the gene revising (gene editing), since the efficiency of Cas9 is affected by local chromatin accessibility due to a significant difference in the activity of gRNA targeting an open genomic region rather than a close genomic region, it is known that there is a positive correlation between the chromatin accessibility and CRISPR-Cas9 mediated gene editing efficiency.

As another aspect of the present disclosure, the present disclosure provides a method for gene editing including bringing the gene editing composition into contact with a target region including a target nucleic acid sequence *in vitro* or ex vivo.

The gene editing composition may be applied to preferably eukaryotic cells, and the eukaryotic cells may be derived from preferably mammals including primates such as humans and rodents such as mice, but are not limited thereto.

As yet another aspect of the present disclosure, the present disclosure provides a gene editing kit including the gene editing composition.

In the present disclosure, the kit may include both a buffer and a material (reagent) required for performing gene editing such as deoxyribonucleotide-5-triphosphate (dNTP) together with the gene editing composition. In addition, an optimal amount of the reagent used in a specific reaction of the kit may be easily determined by those skilled in the art who have learned the disclosure herein.

As still yet another aspect of the present disclosure, the present disclosure provides a method for construction of a gene-modified mammal other than human, including introducing the gene editing composition into a mammal cell other than human to obtain a gene-modified mammal cell; and transplanting the obtained gene-modified mammal cell into the fallopian tube of a mammal foster mother other than human.

The gene editing composition of the present disclosure may be introduced to achieve gene editing efficiency (e.g., base substitution, insertion or deletion) of 40% or more, 45% or more, 50% or more, 55% or more, 60% or more, 65% or more, 70% or more, 75% or more, 80% or more, 85% or more, 90% or more, 95% or more, 97% or more, 99% or more or 100% in the mammal cell.

In the present disclosure, the introducing of the gene editing composition into the mammal cell may be performed by i) transfecting the cell with a plasmid vector or viral vector encoding the prime editing fusion protein according to the present disclosure, pegRNA, nsgRNA, and dsgRNA,
ii) directly injecting a mixture of mRNA encoding the fusion protein, pegRNA, nsgRNA, and dsgRNA or each thereof into the cell, or
iii) directly injecting the mixture of the fusion protein, pegRNA, nsgRNA, and dsgRNA, or a ribonucleic acid protein in the form of a complex into the cell.

The direct injection may mean that each mRNA of ii) or iii) and the guide RNA or the ribonucleic acid protein passes through a cell membrane and/or nuclear membrane to be transferred to the genome without using a recombinant vector, and may be performed by, for example, nanoparticles, electroporation, lipofection, microinjection, and the like.

The mammal cells into which the gene editing composition is introduced may be embryos of mammals including primates such as humans and rodents such as mice, and preferably embryos of mammals other than humans. For example, the embryo may be a fertilized embryo obtained by mating superovulation-induced female mammal and male mammal collected from the fallopian tube of the female mammal. The embryo to which the base revising composition is applied (injected) may be a fertilized 1-cell stage embryo (zygote).

The obtained gene-modified mammal cell may be a cell in which a base substitution, insertion or deletion mutation has occurred in a target gene by introduction of the gene editing composition.

The mammal to which the gene-modified mammal cell, preferably the gene-modified embryonic cell is transplanted into the fallopian tube may be a mammal (foster mother) of the same species as the mammal from which the embryonic cell is derived.

In addition, the present disclosure provides a gene-modified mammal constructed by the method.

Hereinafter, preferred Embodiments will be proposed in order to help in understanding of the present disclosure. However, the following Embodiments are just provided to more easily understand the present disclosure, and the contents of the present disclosure are not limited by the following Embodiments.

### [Embodiments]

### Embodiment 1. Experiment method

### 1-1. Construction of plasmid DNA

High-mobility group nucleosome binding domain 1 (HN1) and histone H1 central globular domain (H1G) oligos were fused on both sides of nCas9 in pCMV-PE2 (#132775, Addgene) using NEBuilder^{®}HiFi DNA Assembly Master Mix (E2621L, NEB) to construct a chromatin-modulating peptide prime editor according to a manufacturer's protocol.

In addition, in order to construct a pegRNA expression vector for inducing specific mutations in *Igf2, Adamts20, Casp1,* and *Hoxd13* genes, in a pU6-pegRNA-GG-receptor vector (#132777, Addgene), spacer, prime binding site and reverse transcriptase template oligos sequences were inserted into a Bsal enzyme cleavage site. The nsgRNA and dsgRNA expression vectors were inserted into a pRG2-GG vector (#104174, Addgene). Additionally, the sequences of pegRNAs, nsgRNAs, and dsgRNAs specific to each target gene used in Embodiment were summarized in Tables 1 to 3 below.

**[Table 1]**

| pegRNA | Sequence (5' to 3') | | PBS length (nt) | RT template length (nt) | SEQ m NO: |
|---|---|---|---|---|---|
| tdTomato_G to C / T ins_8-17 | Spacer | CGCATGGAGGGCTCCATGAA | 8 | 17 | 3 |
| | Primer Binding Site | ATGGAGCC | | | 4 |
| | RT template | GAACTCAGTGGCGGTIC | | | 5 |
| *Igf2_*G to C / TA ins_9-14 | Spacer | TATTGGAAGAACTTGCCCAC | 9 | 14 | 6 |
| | Primer Binding Site | GGCAAGTTC | | | 7 |
| | RT template | AGATACCGCGTGTA | | | 3 |
| *Adamts20_*CG to AA_11-13 | Spacer | AGTGAATAAGAAGACGTACT | 11 | 13 | 9 |
| | Primer Binding Site | ACGTCTTCTTA | | | 10 |
| | RT template | GACCGGCCTTAGT | | | 11 |
| *Casp1*_TAGG del_12-12 | Spacer | GTCTTGTCTCTTATAGGAGA | 12 | 12 | 12 |
| | Primer Binding Site | CCTATAAGAGAC | | | 13 14 |
| | RT template | ACCTCTTTCACT | | | |
| *Hoxd13_*G to T_10-15 | Spacer | GAGGCATACATCTCCATGGA | 10 | 15 | 15 |
| | Primer Binding Site | ATGGAGATGT | | | 16 |
| | RT template | GACTGGTAGACCTCC | | | 17 |
| *Angpt1*_CGG to TGA_11-13 | Spacer | CACATTGCCCATGTTGAATC | 11 | 13 | 18 |
| | Primer Binding Site | TCAACATGGGC | | | 19 |
| | RT template | GATATAACTGAAT | | | 20 |
| *Ksr2*_TGAT ins_8-14 | Spacer | TCCTGCCCTGGCTCCGTGGT | 8 | 14 | 21 |
| | Primer Binding Site | ACGGAGCC | | | 22 |
| | RT template | GTGGCCCACCTGAT | | | 23 |
| *Ar*_G to T_13-13 | Spacer | TCTCACTTGTGGCAGCTGCA | 13 | 13 | 24 |
| | Primer Binding Site | AGCTGCCACAAGT | | | 25 |
| | RT template | AAGAAGACCTTGA | | | 26 |

**[Table 2]**

| Nicking sgRNA | Sequence (5' to 3') | SEQ ID NO: |
|---|---|---|
| tdTomato-nsgRNA | GCCCTCGATCTCGAACTCAG | 29 |
| *Igf2-*nsgRNA | CTTCCCCAGATACCGCGTGT | 28 |
| *Adamt20*-nsgRNA | AGCTCGTGTTCAAGGACATG | 29 |
| *Casp1*-nsgRNA | CTGTCAGAAGTCTTGTGCTC | 30 |
| *Hoxd13-*nsgRNA | GATCCTTGGCACAGTACACC | 31 |
| *Angpt1-*nsgRNA | GATATAACTGAATTCAACAT | 33 |
| *Ksr2*-nsgRNA | ACCTGATACGGAGCCAGGGC | 33 |
| *Ar*-nsgRNA | AGGGGAAAATATCAGGAAGT | 34 |

**[Table 3]**

| dsgRNA | Sequence (5' to 3') | SEQ ID NO: |
|---|---|---|
| *Igf2*_dsgRNA_-34 | AGTCAGCAGGTTTC | 35 |
| *Igf2_dsgRNA_-27* | AGGTGCTAGTCAGC | 36 |
| *Igf2_*dsgRNA_+7 | TGGAGACAGTCCGC | 37 |
| *Igf2*_dsgRNA_+22 | GGACGCCTGCGCAG | 38 |
| *Adamts20*_dsgRNA_-13 | TCAAGAGCACAGCC | 39 |
| *Adamts20_*dsgRNA_+ 14 | TTTCTGGTGCTTGG | 40 |
| *Adamts20_*dsgRNA_+24 | CGATTTTGACTTTC | 41 |
| *Adamts20_*dsgRNA_+41 | CTTCCGCTCACTTC | 42 |
| *Casp1*_dsgRNA_-13 | CTTTGACTTCTCTA | 43 |
| *Casp1*_dsgRNA_-12 | ACTTTGACTTCTCTA | 44 |
| *Casp1*_dsgRNA_+7 | CAGCAAATTCTTTC | 45 |
| *Casp1*_dsgRNA_+62 | GTATTCATGTCTCA | 46 |
| *Hoxd13*_dsgRNA_-12 | CCCGGATCCAAAAG | 47 |
| *Hoxd13_*dsgRNA_-11 | CACTTTTGGATCCG | 48 |
| *Hoxd13_*dsgRNA_+20 | GCTGTTCCACCCGT | 49 |
| *Hard13*_dsgRNA_+24 | CGGGTGGAACAGCC | 50 |
| *Angpt1_*dsgRNA_-15 | CGAAATCCAGAAAA | 51 |
| *Angpt1*_dsgRNA_-11 | ATCCAGAAAACGGA | 52 |
| *Angpt1_*dsgRNA_+14 | CTTCCAGAACACGA | 53 |
| *Angpt1_*dsgRNA_+17 | TTCCCGTCGTGTTC | 54 |
| *Ksr2*_dsgRNA_-12 | TCTCCAAACAAGAT | 55 |
| *Ksr2_*dsgRNA_+7 | TCCGGGGGGCACAC | 56 |
| *Ar*_dsgRNA_-15 | GGAGATGAAGCTTC | 57 |

### 1-2. Lipofection and Electroporation

HEK293T cells, NIH/3T3 cells (ATCC, CRL-1658) and C2C12 cells (ATCC, CRL-1772) introduced with a reporter system expressing tdTomato at AAVS1 loci were incubated in a Dulbecco's modified Eagle's medium (DMEM; LM001-05, Welgene) supplemented with 10% FBS (S 001-01, Welgene) or BCS (26170-043, Gibco) under conditions of 5% CO₂ and 37°C. In Embodiment, 2 × 10⁴ cells were treated and transfected with 50 µl of Opti-MEM (31985070, Gibco) containing three plasmids of 0.5 µg pegRNA, 2.15 µg PE2, and 0.22 µg nsgRNA, and 1 µl of a Lipofectamine 2000 reagent (11668019, Thermo Fisher Scientific) according to a manufacturer's protocol, and then the cells were incubated for 11 days.

Electroporation was performed on 1 × 10⁵ NIH/3T3 cells and C2C12 cells, respectively, and each of the cell lines was mixed with plasmids of 3 µg PE2 or CMP-PE, 0.7 µg pegRNA, 0.3 µg nsgRNA and 0.25 µg dsgRNA, and transfected using the Neon^{™} Transfection System (MPK1096, Thermo Fisher Scientific) according to a manufacturer's protocol. Thereafter, the cells were incubated for 72 hours, and then recovered and subjected to targeted deep sequencing.

### 1-3. Preparation of DNA amplicons

After transfection, genomic DNA was extracted from tdTomato expressing reporter HEK293T, NIH/3T3, C2C12 cells and mouse embryos using DNeasy Blood & Tissue Kits (69506, Qiagen). Thereafter, the edited target sequence was amplified using Phusion^{™} High-Fidelity DNA polymerase (F-530XL, Thermo Fisher Scientific) and Sungen (SG-PT02, Sun genetics).

### 1-4. In vitro transcription

Transcripts of PE2 and CMP-PE were prepared using a mMESSAGE mMACHINE T7 Ultra Kit (AM1345, Invitrogen) and purified using a MEGAclear^{™} Transcription Clean-Up Kit (AM1908, Invitrogen). Transcription of pegRNA, nsgRNA, and dsgRNA was induced using T7 RNA polymerase (M0251, NEB) according to the manufacturer's protocol, and transcribed RNAs were purified using ExpinTM CleanUp SV (113-150, GeneAll). Thereafter, the purified RNAs were quantified using NanoDrop One UV-Vis (Thermo Fisher Scientific).

### 1-5. Experimental Animals

An *in vivo* experiment of Embodiment was conducted with the approval of the Institutional Animal Care and Use Committee (IACUC) of Seoul National University. C57BL/6N and ICR mice were used for embryo donation, and surrogate mothers were bred in a specific pathogen-free laboratory.

### 1-6. Microinjection

HyperOva (KYD-010-EX-x5, CARD) and hCG (CG10-1vl, Sigma) were injected into C57BL/6N female mice every 48 hours to induce ovarian hyperstimulation, and injected with a hCG hormone, and then female mice were mated with wild-type C57BL/6N male mice. Embryos at the fertilized one-cell stage were obtained from the ampulla and incubated in a M2 medium (M7167, Sigma) until two pronuclei appeared. A prime editing mixture for microinjection was prepared at concentrations of 100 ng of PE2 or CMP-PE mRNA, 65 ng pegRNA, 32.5 ng nsgRNA, and 22 ng dsgRNA in 100 µl of Tris-EDTA (pH 7.4). After microinjection, the embryos were incubated for 4 days in a 37°C incubator under a KSOM medium (MR-121-D, Millipore) for development into blastocysts. Thereafter, some of two-cell stage embryos were transplanted to the fallopian tube of a pseudopregnant mother.

### 1-7. Genotyping and targeted deep sequencing

Each target was amplified by nested PCR using specific primers. A library consisting of PCR amplicons was subjected to sequencing using the iSeq^{™} 100 sequencing system (Illumina, Inc.). Thereafter, sequencing data was analyzed through a CRISPR REGN Tools program (http://www.rgenome.net/) and a EUN program (https://daeunyoon.com/).

### 1-8. Whole genome sequencing and variant calling

Genomic DNA was isolated from the ears of mice (C57BL/6N) using a DNeasy Blood & Tissue kit (69506, Qiagen) and then cleaved using a Covaris S2 ultrasonic device system according to the manufacturer's instructions to construct a paired-end DNA library using a Truseq Nano DNA sample prep kit. Deep coverage (30x) full-length genome sequencing was performed on an Illumina Novaseq 6000 platform (Illumina) through 101 base paired-end sequencing, and sequence reads were performed using BWA-MEM to align the mouse reference genome GRCm38/mm10. Single nucleotide mutation and small insertion/deletion (indel) were detected using GATK4 HaplotypeCaller, and known mutations present in dbSNP for mouse v142 (dbSNP142) were annotated with ANNOVAR. New variants not present in dbSNP142 were further analyzed to confirm their locations in an off-target region. The estimated off-target site was compared with the candidate of Cas-OFFinder considering the maximum 7-bp or 2-bp bulge + 5 bp mismatch. All variants were manually identified by visualizing a readout plot.

### 1-9. DNase I digestion assay and qPCR

The present inventors performed a DNase I digestion assay according to a conventionally known method. Specifically, the cells were detached, washed repeatedly with cold 1X PBS, and then spun-down twice for 5 minutes at 900 rpm. Then, the cells were lysed using a cold RSB buffer (10 mM Tris-HCl, 10 mM NaCl and 3 mM MgCl₂) + 0.1% IGEPAL CA-630 (I8896, Sigma)) and spun-down for 10 minutes at 4deg to pellet nuclei. Next, a supernatant was removed, and the nuclei were incubated at 37°C for 20 to 30 minutes with or without DNase I (2 to 16 U) treatment. Thereafter, the reaction was stopped by adding 50 mM EDTA, and DNA lysed with DNase I was purified using a DNeasy Blood & Tissue kit (69506, Qiagen). Real-time qPCR was performed using a KAPA SYBR FAST qPCR Master Mix Kit (KR0389, Kapa Biosystems), and the fraction of intact genomic DNA was measured using a comparative CT method. Primer sequences used in the real-time qPCR were listed in Table 4 below.

**[Table 4]**

| Target | Sequence (5' to 3') | | SEQ ID NO: |
|---|---|---|---|
| Closed chromatin | Forward | GCAGCAGATGGCAAGTAATACTAAGAT | 58 |
| | Reverse | CCCTTATTCTCTGAGCATTAGACAGTTATA | 59 |
| Open chromatin | Forward | TGAGTCACAGCATCAGCATCGGGTCTCT | 60 |
| | Reverse | GGAGAGGGCCTTTTTCTCTTCAAGGTTC | 61 |
| *Igf2* | Forward | TCTCCAGGACGACTTCCCCAGA | 62 |
| | Reverse | CTCCAGGTGTCATATTGGAAGAAC | 63 |
| *Adamts20* | Forward | GTGGAATCAAGAGCACAGC | 64 |
| | Reverse | CAGTGAATAAGAAGACGTAC | 65 |
| *Casp1* | Forward | AAATCACTGGTCTTGTCTCTTATAG | 66 |
| | Reverse | CAGCAAATTCTTTCACCTCTTTC | 67 |
| *Hoxd13* | Forward | TCGGCACGAGGCATACATCTCCATG | 68 |
| | Reverse | CGTTGGCTAGCGTCCAGGACTG | 69 |
| *Angpt1* | Forward | CCAGAAAACGGAGGGAGAAGA | 70 |
| | Reverse | TAGGCACATTGCCCATGTTGA | 71 |
| *Ksr2* | Forward | TCTCTCCAAACAAGATTGGATCAT | 72 |
| | Reverse | TCTCCTGCCCTGGCTCC | 73 |

### 1-10. Statistical analysis

Data were presented as mean and standard deviation, and 3 or 5-time independent repeated experiments were performed. P values were calculated using unpaired and two-sided Student's t-test.

### Embodiment 2. Verification of prime editing system in mammal genome and confirmation of editing efficiency

The present inventors first tried to verify the applicability of a prime editing system to a mammal genome, and for this purpose, a reporter system expressing tdTomato was used in an AAVS1 locus of HEK293T cells. Specifically, as illustrated in FIG. 1A, a stop codon was generated by inserting a thymine (T) base into the tdTomato sequence using a prime editor 3 (PE3) having a primer binding site (PBS) length of 8 nt and a reverse transcriptase (RT) template length of 17 nt (PBS8-RT17). In addition, prime-editing guide RNA (pegRNA) was designed to remove a PAM sequence on a non-target strand to inhibit editing on the edited strand. Thereafter, the present inventors evaluated the editing efficiency by gating tdTomato-negative cells through flow cytometry to confirm the editing efficiency of the tdTomato gene by the PE3.

As a result, as illustrated in FIG. 1B, it was confirmed that when compared with a positive control (tdTomato expressing HEK293T) expressing tdTomato, tdTomato gene editing was induced using PE3 (tdTomato expressing HEK293T + PE3), 32% of cells were tdTomato-negative. These results suggested that the prime editor enables the creation of desired target mutations in the system of the present disclosure.

Next, in order to construct a mouse model using the prime editor, it was tried to design a target containing 8 transitional mutations or one or more nucleotide insertions or deletions. First, as illustrated in FIG. 2A, the generation of stop codons was induced in two mouse genes, that is, insulin-like growth factor 2 (*Igf2*) and a disintegrin and metalloproteinase domain with thrombospondin type-1 motifs 20 (*Adamts20*)*.* The *Igf2* gene may induce a dwarfism phenotype by mutation of a *Igf2* allele inherited from the father. Specifically, the present inventors generated a stop codon by inserting a TA base into exon 4 of the *Igf2* gene to induce loss of function of the gene. In addition, nucleotides of the PAM sequence were substituted from NGG to NCG to suppress continuous editing of the edited strand. Meanwhile, *Adamts20* was a gene involved in the development of melanocytes, and it was known that the generation of a premature stop codon at an E584 site of an *Adamts20* locus was associated with a typical white belt phenotype. The present inventors induced the premature stop codon (E584^{∗}) and modification (from NGG to NAG) of the PAM sequence by inducing substitution of CG with TT in exon12 of the *Adamts20* locus. In addition, the present inventors used the PE3 system consisting of PE (nCas9 fused with engineered M-MLV RT), pegRNA and nicking sgRNA (nsgRNA) to induce the mutations in these mouse gene target. At this time, the nsgRNA was used for the purpose of improving the editing efficiency by promoting DNA repair activity through cleavage of the target DNA strand, that is, the non-editing strand.

Next, the present inventors evaluated the editing efficiency according to pegRNAs consisting of various PBS lengths (8 to14 nt) and RT template lengths (10 to 18 nt) to optimize prime editing at *Igf2* and *Adamts20* target sites. At this time, the length of PBS with thymine at a 3'-end, which may be a part of a transcription termination signal, and the length of RT with cytosine at a 5'-end, which may interfere with the pegRNA structure, were excluded. Thereafter, three plasmids encoding PE, pegRNA, and nsgRNA were transfected into NIH/3T3 cells by electroporation, and the cells were incubated for 72 hours, collected, and subjected to targeted deep sequencing.

As a result of the experiment, as illustrated in FIG. 2B, it was confirmed that the PE3-mediated editing efficiency for *Igf2* and *Adamts20* targets in NIH/3T3 cells was less than 3%. From these results, it was determined that the editing efficiency needs to be improved in order to use the prime editor for construction of the mouse model.

### Embodiment 3. Confirmation of enhanced editing efficiency of prime editor by dsgRNA

As an attempt to improve the editing efficiency of the prime editor, the present inventors used dsgRNAs to release a chromatin structure of a target site instead of catalytically dead endonuclease based on a proxy-CRISPR idea. The dsgRNA was guide RNA having a length of 14 to 15 nt that guided the Cas endonuclease to bind to the target site while exhibiting deactivated catalytic activity. Accordingly, the present inventors assumed that the prime editor would play the following two roles. One was a prime editing function with pegRNA at the target site, and the other was modulating dsgRNA and chromatin adjacent to the target site. The present inventors designed proximal dsgRNAs adjacent to *Igf2* and *Adamts20* target sites in the range of locations of 7 to 62 nucleotides from the spacer of pegRNA. Thereafter, in order to confirm the editing efficiency, proximal dsgRNA was applied to various pegRNA lengths at the *Igf2* and *Adamts20* sites. Interestingly, it was shown that the editing efficiency of PE3 using proximal dsgRNA improved in most groups. Therefore, the present inventors performed subsequent experiments by selecting PBS9-RT14 pegRNA and PBS11-RT13 pegRNA, which showed the highest efficiency against *Igf2* and *Adamts20* targets, respectively.

Next, in order to select dsgRNAs with high editing efficiency, additional proximal dsgRNAs for the genes *Igf2, Adamts20, Casp1* (4 bp deletion), *Hoxd13* (G to T substitution), *Angpt1* (CGG to TGA substitution) and *Ksr2* (TGAT insertion) genes were designed and tested. To this end, plasmids encoding PE, pegRNA, nsgRNA and each proximal dsgRNA were transfected into NIH/3T3 and C2C12 cells by electroporation, and then subjected to targeted deep sequencing.

As a result, as can be seen in FIG. 3, it was confirmed that the proximal dsgRNA selectively improved the editing efficiency in most targets compared to PE3. From these results, it can be seen that the gene editing efficiency according to dsgRNA application varies depending on the location of proximal dsgRNA, and a screening process for the optimal dsgRNA for each target and cell type is required to induce effective target mutation.

### Embodiment 4. Confirmation of enhanced editing efficiency of prime editor bound with CMP

As another attempt to improve the editing efficiency of the prime editor, the present inventors constructed the prime editor using the high-mobility group nucleosome binding domain 1 (HN1) and the histone H1 central globular domain (H1G) as chromatin-modulating peptides (CMPs). Specifically, two versions were prepared by adding the HN1 and the H1G to the protein of the prime editor in which the Cas9 nickase (nCas9) and the reverse transcriptase were fused. As illustrated in FIG. 4A, a fusion protein consisting of the HN1 bound to the N-terminus of nCas9 and the H1G bound to the C-terminus was referred to as CMP-PE-V1, and a fusion protein constructed so that the HN1 was bound to the N-terminus of nCas9, and the H1G was bound to the M-MLV RT constructed to the C-terminus of nCas9 and the C-terminus of the RT was referred to as CMP-PE-V2. In addition, the amino acid sequences of CMP-PE-V1 and CMP-PE-V2 were illustrated in FIGS. 4B and 4C, respectively.

The present inventors delivered the CMP-PE3-V1 (pegRNA/nsgRNA and CMP-PE-V1) or the CMP-PE3-V2 (pegRNA/nsgRNA and CMP-PE-V2) to two mouse cell lines, respectively, and compared the editing efficiency with that of a case of introducing PE3 without binding the CMP into the cells. As a result, as illustrated in FIG. 4B, it was confirmed that the editing efficiency of CMP-PE3-V1 was much higher than that of PE3 at most of the target sites. In particular, the editing efficiency by CMP-PE3-V1 was 2.55 times higher than *Igf2* and 3.92 times higher than *Adamts20* in NIH/3T3 cells. In addition, the editing efficiency was confirmed by applying the enhanced prime editor and dead sgRNA targeting the HEK3 sequence in human HEK293T cells. The result was illustrated in FIG. 4E. It was confirmed that the editing efficiency was improved in CMP-PE-V1 or CMP-PE-V2, which were enhanced prime editing methods compared to PE3. In addition, when the dead sgRNA was applied together with the enhanced prime editor, it was confirmed that the efficiency was improved more than when only PE3 or the enhanced editor was treated (CMP-PE3-V2 + dsgRNA(-11). These results show that the prime editor engineered using the chromatin-modulating peptides HN1 and H1G can significantly enhance editing efficiency.

### Embodiment 5. Confirmation of effect of enhancing editing efficiency of prime editor applied with dsgRNA and CMP

Furthermore, based on the results of Embodiments 3 and 4, the present inventors analyzed whether a synergistic effect on editing efficiency appeared by delivering CMP-PE3-V1 and dsgRNA (CMP-PE3-V1 + dsgRNA) together to mouse cells.

As a result, as illustrated in FIG. 5, when CMP-PE3-V1 and dsgRNA were delivered together to mouse cells (CMP-PE3-V1 + dsgRNA), compared to the PE3 control, the editing efficiency was up to 4.20-fold, 5.11-fold, and 3.56-fold enhanced at *Igf2, Adamts20,* and *Hoxd13* target sites, respectively, and these synergistic effects appeared at different levels depending on a cell line and a target. In addition, from the results, it can be seen that when only CMP-PE3 was introduced into cells (CMP-PE3-V1), the efficiency of the prime editor was significantly enhanced in all targets and two cell types to be experimented, whereas when only dsgRNA was introduced into cells (PE3 + dsgRNA) or when CMP-PE3-V1 and dsgRNA were introduced together (CMP-PE3 + dsgRNA), the editing efficiency could vary depending on a target site and a cell type.

Furthermore, the present inventors attempted to induce targeted mutagenesis by injecting the advanced prime editor system into mouse embryos through microinjection and analyze its efficiency. Specifically, among the designed mouse targets, an *Igf2* target site with relatively low undesired mutations was selected. As a result, as illustrated in FIG. 6A, the embryos injected with CMP-PE3-V1 and the embryos injected with CMP-PE3-V1 + dsgRNA showed a significantly high level of editing efficiency for the *Igf2* target. In particular, it was confirmed that in the case of CMP-PE3-V1 + dsgRNA, the desired mutations at the target site of the *Igf2* gene were observed in 21 of 22 embryos (95%), so that CMP-PE3-V1 + dsgRNA exhibited significantly higher the editing efficiency than PE3 and PE3 + dsgRNA.

In addition, the CMP-PE-V1 and proximal dsgRNA were used and tested to verify the editing efficiency in the *Adamts20, Hoxd13, Angpt1, Ksr2,* and Ar genes in mouse embryos. As a result of targeted deep sequencing, as illustrated in FIG. 6B, it was confirmed that the editing efficiency was improved at *Adamts20, Hoxd13,* and *Angpt1* targets, except for *Ksr2* and Ar targets, in embryos injected with CMP-PE-V1 and proximal dsgRNA compared to those injected with PE3. Collectively, these results suggest that the prime editing with enhanced editing efficiency is possible using proximal dsgRNA and chromatin-modulating peptides in mouse embryos.

### Embodiment 6. Confirmation of enhanced chromatin accessibility of target site by dsgRNA and CMP

In order to confirm whether CMP-PE-V1 and dsgRNA can improve chromatin accessibility by releasing the chromatin structure of the target site, the present inventors confirmed the chromatin state of each target site by performing DNaseI digestion analysis and qPCR. At this time, a gene located at Chr 3: 71,026,628-71,026,685 (mouse genome build mm9) was used as a negative control (closed chromatin), and a Co16a1 gene was used as a positive control (open chromatin). As a result of the experiment, as illustrated in FIGS. 7A and 7B, it was analyzed that *Igf2, Adamts20,* and *Hoxd13* appeared as relatively closed chromatin in the NIH/3T3 cell line, and in C2C12, all targets except for *Igf2* had open chromatin. These results suggest that the state of the chromatin structure is different in the two cell lines even though the target sequences are identical.

Furthermore, it was analyzed whether CMP-PE-V1 or dsgRNA could change the chromatin state at the target site of *Igf2,* a representative gene identified as having a closed chromatin structure. As a result, as can be seen in FIG. 7C, it was confirmed that the chromatin structure in the closed state was gradually changed to the open state by CMP-PE-V1, dsgRNA or CMP-PE-V1 + dsgRNA when compared to PE3. These results are direct evidence that the efficiency of prime editing may be enhanced by releasing the closed chromatin structure and improving chromatin accessibility using CMP-PE-V1 and dsgRNA.

### Embodiment 7. Construction of mouse model with targeted mutations and analysis of off-target effect

Next, the present inventors induced the generation of targeted mutations of *Igf2* as illustrated in FIG. 8A in mouse embryos through microinjection using PBS9-RT14 and dsgRNA +7, which had relatively low frequencies of undesired mutations, and transplanted mouse embryos into a surrogate mother. Thereafter, as a result of observing and analyzing young born from the surrogate mother, as shown in FIG. 8B, it was confirmed that G to C substitution and TA insertion occurred at the *Igf2* locus with an editing frequency (2 out of 10 mice) of up to 47%. In addition, as a result of analyzing whether the *Igf2* mutation was transmitted to the next generation, as shown in FIG. 8C, it could be seen that 7 out of 9 F1 littermates born from *Igf2* mutant mice had the same mutation, so as to be transmitted to the next generation through germ cells of the target mutation.

To evaluate the off-target effect of the prime editor, the present inventors used Cas-OFFinder to confirm potential off-targets by pegRNAs and nsgRNAs of *Igf2* targets with up to three nucleotide mismatches each in the mouse genome. As a result, as illustrated in FIG. 9A, no potential off-target mutation was detected when compared to the wild type. In addition, whole genome sequencing (WGS) was performed to confirm the off-target effect in the constructed *Igf2* mutant mice. As a result, as illustrated in FIGS. 9B and 9C, a single off-target site of nsgRNA was found, but through Sanger sequencing using genomic DNA, it was confirmed that the site was a false positive.

Finally, in order to confirm the phenotypes of *Igf2* mutant mice, *Igf2*^{p+/m-} males (F1) were mated with wild-type female mice. As a result, as illustrated in FIGS. 10A and 10B, *Igf2*^{p-/m+} mice with a mutation of the *Igf2* gene inherited from a paternal allele showed a dwarfism phenotype consistent with a desired mutant genotype. These results suggest that the enhanced prime editing system according to the present disclosure can be effectively applied to the construction of the mouse model.

The aforementioned description of the present disclosure is used for exemplification, and it can be understood by those skilled in the art that the present disclosure can be easily modified in other detailed forms without changing the technical spirit or requisite features of the present disclosure. Therefore, it should be appreciated that the embodiments described above are illustrative in all aspects and are not restricted.

## Claims

1. A gene editing composition comprising:
(a) a fusion protein or a nucleic acid encoding the fusion protein, including i) a CRISPR/Cas9 protein or a variant thereof, and ii) a reverse transcriptase or a variant thereof; and
(b) a guide RNA or a nucleic acid encoding the guide RNA,
wherein the guide RNA includes prime editing guide RNA (pegRNA) and dead single guide RNA (dsgRNA), and
the dsgRNA is 10 to 20 nucleotides (nt) in length.

2. The gene editing composition of claim 1, wherein the dsgRNA binds to a location of 5 to 70 nt away from a pegRNA binding site to increase chromatin accessibility of the fusion protein.

3. The gene editing composition of claim 1, further comprising:
single guide RNA (sgRNA) that complementarily binds to a non-target DNA strand to induce cleavage of a target DNA strand.

4. A gene editing composition comprising:
(a) a fusion protein or a nucleic acid encoding the fusion protein, including i) a CRISPR/Cas9 protein or a variant thereof, ii) a reverse transcriptase or a variant thereof, and iii) chromatin-modulating peptides; and
(b) a guide RNA or a nucleic acid encoding the guide RNA,
wherein the guide RNA includes prime editing guide RNA (pegRNA) and dead single guide RNA (dsgRNA).

5. The gene editing composition of claim 4, wherein the chromatin-modulating peptide is a high-mobility group nucleosome binding domain 1 (HN1), a histone H1 central globular domain (H1G), or a combination thereof.

6. The gene editing composition of claim 4, wherein the chromatin-modulating peptide is linked to the CRISPR/Cas9 protein or the reverse transcriptase directly by a chemical bond, indirectly by a linker, or in combination thereof.

7. The gene editing composition of claim 4 or 5, wherein the fusion protein consists of N-terminus-[HN1]-[Cas9]-[H1G]-[reverse transcriptase]-C-terminus; or N-terminus-[HN1]-[Cas9]-[reverse transcriptase]-[H1G]-C-terminus.

8. The gene editing composition of claim 4 or 5, wherein the fusion protein further includes a nuclear localization signal (NLS) sequence at a N-terminus and C-terminus, respectively.

9. The gene editing composition of claim 4, wherein the CRISPR/Cas9 protein variant is nickase.

10. The gene editing composition of claim 9, wherein in the CRISPR/Cas9 protein variant, either a RuvC domain or an HNH domain is deactivated.

11. The gene editing composition of claim 4, wherein the reverse transcriptase or the variant thereof is derived from a Moloney murine leukemia virus (M-MLV).

12. The gene editing composition of claim 4, wherein the fusion protein consists of an amino acid sequence represented by SEQ ID NO: 1 or SEQ ID NO: 2.

13. The gene editing composition of claim 4, wherein the dsgRNA consists of 10 to 20 nucleotides (nt) in length.

14. The gene editing composition of claim 4, wherein the dsgRNA binds to a location of 5 to 70 nt away from a pegRNA binding site to increase chromatin accessibility of the fusion protein.

15. The gene editing composition of claim 4, further comprising:
single guide RNA (sgRNA) that complementarily binds to a non-target DNA strand to induce cleavage of a target DNA strand.

16. The gene editing composition of claim 1 or 4, wherein the composition enhances a gene editing efficiency and target specificity.

17. A method for gene editing comprising bringing the gene editing composition of claim 1 or 4 into contact with a target region including a target nucleic acid sequence *in vitro* or *ex vivo.*

18. A gene editing kit comprising the gene editing composition of claim 1 or 4.

19. A method for construction of a gene-modified mammal other than human comprising: obtaining a gene-modified mammal cell by introducing the gene editing composition of claim 1 or 4 into a mammal cell other than human; and
transplanting the obtained gene-modified mammal cell into a fallopian tube of a mammal foster mother other than human.

20. The method for construction of claim 19, wherein the mammal cell is a mammal embryonic cell.
